# EUROPÄISCHE PATENTSCHRIFT

(11) **EP 2 710 360 B1**
(45) Veröffentlichungstag und Bekanntmachung des Hinweises auf die Patenterteilung: **12.02.2020**
(21) Anmeldenummer: 12720809.8
(22) Anmeldetag: 11.05.2012
(51) Int. Cl.: G01N 27/22, G01N 27/07, G01K 1/14, G01K 13/02, G01N 33/28

(54) **Vorrichtung zur Aufnahme und/oder Leitung eines flüssigen oder gasförmigen Mediums und Verfahren zur Herstellung einer derartigen Vorrichtung**
Device for receiving and/or conducting a liquid or gaseous medium and method for producing such a device
Dispositif pour recevoir et/ou conduire un milieu liquide ou gazeux et procédé pour produire un tel dispositif

(30) Priorität: 16.05.2011 DE 102011101503
(43) Veröffentlichungstag der Anmeldung: 26.03.2014
(73) Patentinhaber: Schott AG, 55122 Mainz (DE)
(72) Erfinder: RANFTL, Reinhard, 84076 Pfeffenhausen (DE); HARTL, Helmut, A-1210 Wien (AT)
(74) Vertreter: Sawodny, Michael-Wolfgang
(86) Internationale Anmeldenummer: PCT/EP2012/002033
(87) Internationale Veröffentlichungsnummer: WO 2012/156057

(56) Entgegenhaltungen:
- US-A1- 2005 121 323
- US-A1- 2007 056 365
- US-A1- 2009 008 250
- US-A1- 2010 156 443
- US-A1- 2010 186 501

## Beschreibung

Die Erfindung betrifft eine Vorrichtung zur Aufnahme und/oder Leitung eines flüssigen oder gasförmigen Mediums umfassend einen Rohrabschnitt mit einer Rohrwandung, wobei die Rohrwandung eine Öffnung und der Rohrabschnitt einen Durchmesser und ein erstes und ein zweites Ende aufweist sowie ein Verfahren zur Herstellung einer derartigen Vorrichtung.

Im Stand der Technik werden verschiedene Arten von Sensoren, beispielsweise Temperatursensoren, verwandt, um die physikalischen Eigenschaften eines Mediums, beispielsweise einer Flüssigkeit oder eines Gases, zu erfassen. Das Medium, dessen physikalische Eigenschaften, beispielsweise die Temperatur, sensiert werden sollen, wird beispielsweise in einem Leitungssystem geführt oder in einem Behälter wie beispielsweise einem Tank aufbewahrt.

Bislang waren die Sensoren zu einer schnellen Erfassung der physikalischen Größen, beispielsweise der Temperatur, auf der Seite der Durchführung angeordnet, die nahe dem Medium, das heißt nahe der Flüssigkeit beziehungsweise des Gases liegt.

Ein Sensor, der derart angeordnet ist, ist dem Medium, das heißt der Flüssigkeit beziehungsweise dem Gas ausgesetzt. Handelt es sich um ein System, in dem ein gewisser Druck aufgebaut wird, so sind die Sensoren auch den Drücken ausgesetzt. Eine derartige Anordnung ist insbesondere problematisch, wenn es sich bei den Medien um aggressive Medien, beispielsweise Säuren, handelt und/oder hohe Drücke in den Systemen herrschen. In solchen Fällen müssen bei einer sogenannten medienseitigen Anordnung der Sensoren auf der Durchführung teure Spezialsensoren eingesetzt werden.

Ein weiterer Nachteil von medienseitig angeordneten Sensoren ist darin zu sehen, dass bei Defekten derartig angeordnete Sensoren nur schwer oder gar nicht zugänglich sind. Auch ein Kalibrieren ist nur mit hohem Aufwand möglich.

Alternativ zu einer medienseitigen Anordnung der Sensoren können Sensoren, beispielsweise Temperatursensoren, auch an der Außenwand eines Gefäßes, beispielsweise einem Tank oder einer Leitung angeordnet werden.

Bei einer Anordnung außerhalb des Mediums ergibt sich jedoch das Problem, dass es zu Messungenauigkeiten kommt. Ein weiteres Problem besteht darin, dass bei einer Anordnung eines Sensors an der Außenwand beispielsweise der Leitung oder des Tankes Temperaturänderungen nur sehr ungenau detektiert werden können. Ein Grund hierfür ist die große thermische Masse der Leitung beziehungsweise des Tankes, die zu einer hohen Trägheit führen und die Detektion von sehr schnell ablaufenden Temperaturwechseln nicht erlauben.

Anstelle der Anordnung auf der Außenwandung der Leitung beziehungsweise des Tankes können die Sensoren auch in einem speziellen Sensorgehäuse in die Leitung eingesetzt werden, wobei der Sensor in dem Sensorgehäuse auf der medienabgewandten Seite angeordnet ist.

Eine derartige Anordnung ist beispielsweise in der US 5,367,264 beschrieben.

Im Wesentlichen dient der aus der US 5,367,264 bekannte Sensor dazu, den Volumenanteil eines Stoffes bei einer Mischung von zwei Stoffen zu bestimmen. Aus der US 5,307,264 ist insbesondere ein Sensor bekannt geworden, mit dem in einem Rohr eine Temperatur eines durchströmenden Mediums gemessen werden kann bzw. dessen genaue Zusammensetzung.

Aus der US 2005/0121323A1 ist ein weiterer Sensor bekannt, mit dem die Konzentration von Additiven oder Verunreinigungen in Öl bestimmt werden sollen. Der Sensor gemäß der US 2005/0121323A1 umfasst ein Gehäuseteil, der so mit einer Leitung verbunden ist, dass eine Elektrode hermetisch dicht von dem Gehäuseteil aufgenommen wird. Eine Temperaturmessung ist in der US 2005/0121323A1 nicht offenbart, auch nicht andere Arten von Sensoren.

Aus der US 2010/0186501 A1 ist eine Vorrichtung mit einem Sensorbauteil bekannt geworden, dass ein rohrförmiges Sensorbauteilgehäuse umfasst. Das Sensorbauteilgehäuse weist eine Rohrwand und wenigstens eine in die Rohrwandung eingelassene Öffnung, in die eine Durchführung für ein Sensorbauteil eingelassen werden kann, auf. Als Sensorbauteil wird insbesondere ein in das Sensorbauteilgehäuse eingelassenes Innenrohr verwandt. Wird das Sensorbauteilgehäuse in seiner Form als Außenrohr auf ein Potential gelegt und das als Sensorbauteil ausgestaltete Innenrohr auf ein anderes Potential, so ist es möglich, eine kapazitive Messung vorzunehmen, die Aufschluss über die Zusammensetzung des durch das Sensorbauteil hindurch tretenden Mediums gestattet.

Hergestellt wird das Sensorbauteilgehäuse gemäß der US 2010/0186501 aus einem runden Rohling durch spanende Bearbeitung, beispielsweise durch Drehen oder Fräsen. Eine Herstellung des Sensorbauteilgehäuses gemäß der US 2010/0186501 A1 mittels Dreh- und Fräsprozessen ist aber sehr aufwändig. Des Weiteren ergeben sich hohe Materialverluste von bis zu 75% vom Rohteil zum Fertigteil. Problematisch ist auch die Ausgestaltung der Rohrenden, und insbesondere ihr Anschluss beispielsweise an Treibstoffleitungen.

Aufgabe der Erfindung ist es somit eine Vorrichtung zur Aufnahme und/oder Leitung eines flüssigen oder gasförmigen Mediums anzugeben, mit der die zuvor beschriebenen Nachteile des Standes der Technik, insbesondere in Form der US 2010/0186501 A1 überwunden werden können. Insbesondere soll ein Sensorbauteilgehäuse angegeben werden, dass sich durch einen minimalen Fertigungsaufwand auszeichnet und sicher an den Enden, beispielsweise an eine weiterführende Leitung, bevorzugt eine Treibstoffleitung angeschlossen werden kann.

Erfindungsgemäß wird diese Aufgabe durch eine Vorrichtung gemäß Anspruch 1 gelöst, die einen Rohrabschnitt mit einer Rohrwandung umfasst, wobei die Rohrwandung eine Öffnung aufweist und der Rohrabschnitt einen Durchmesser und ein erstes und ein zweites Ende. Des Weiteren ist vorgesehen, dass das erste und/oder das zweite Ende Ankippungen, die durch ein Herstellverfahren, das einen Umformschritt umfasst, hergestellt wurde, aufweist, so dass sich der Durchmesser zum ersten und/oder zweiten Ende des Rohrabschnittes hin verjüngt. Eine derartige Ausgestaltung mit einer Ankippung erlaubt es, dass das erfindungsgemäße Sensorbauteilgehäuse auf einfache Art und Weise in einen Leitungsstrang, beispielsweise eine Treibstoffleitung eingesetzt werden kann, wobei durch die Ankippung die Dichtheit des Übergangs von der Leitung zum Sensorbauteilgehäuse gewährleistet wird.

Erfindungsgemäß wird durch die Öffnung des Sensorbauteilgehäuses eine Durchführung für ein Sensorbauteil, ein Innenrohr für eine kapazitive Messung, wie in der US 2010/0186501 A1 detailliert beschrieben, hindurchgeführt.

In einer besonders einfachen Ausführungsform, wird die Durchführung durch die Öffnung hindurchgeführt. Die Durchführung weist ein Verstärkungsbauteil auf, wobei das Verstärkungsbauteil mit dem Sensorbauteilgehäuse durch Hartlöten verbunden wird.

Die Durchführung wird mehrteilig ausgeführt und weist, ein Glas- oder Glaskeramikmaterial und ein Verstärkungsbauteil auf.

Die Verwendung eines Verstärkungsbauteiles hat den Vorteil, dass unterschiedliche Materialien für das Sensorbauteilgehäuse und das Verstärkungsanteil verwandt werden können. Die Materialien für das Sensorbauteilgehäuse und das Verstärkungsbauteil können unterschiedlich gewählt werden und zwar derart, dass die Materialien angepasst und abgestimmt sind. So ist es beispielsweise vorteilhaft, wenn das Sensorbauteilgehäuse auf das durch das Sensorbauteilgehäuse durchströmende Medium, insbesondere das zu detektierende Medium angepasst ist.

Das Verstärkungsbauteil kann hingegen ein Material umfassen, dass sich insbesondere durch eine hohe Festigkeit auszeichnet. Das Material für das Verstärkungsbauteil besteht vorteilhaft aus einem korrosionsbeständigen Material und ist abgestimmt auf eine stabile, sichere optimale Einglasung. Für das Sensorbauteilgehäuse kann ein relativ dünnwandiges Rohrmaterial verwandt werden, was zu Materialeinsparungen gegenüber dem Stand der Technik führt, da ein Abdrehen oder Abfräsen von überschüssigem Material wie z. B. in der US 2010/0186501 beschrieben nicht nötig ist. Die im Bereich der Durchführung für das Sensorbauteil benötigte größere Wandstärke wird nämlich durch das Verstärkungsbauteil zur Verfügung gestellt.

Das Überschieben von an den angekippten Enden angeschlossenen Leitungen wird durch an der Rohrwandung des Sensorbauteilgehäuses vorgesehenen Verdickungen verhindert. Die Verdickungen wirken praktisch als Anschlag für die über die Ankippung auf das Sensorbauteilgehäuse aufgeschobenen Anschlussleitungen.

Die Herstellung der Ankippung umfasst einen Umformprozess, beispielsweise das Umformen durch Stauchen die Herstellung kann alleine durch Umformen, beispielsweise durch Stauchen, erfolgen oder die Umformung wird nach einer Bearbeitung, beispielsweise Anfasen, durch Fräsen durchgeführt. Bevorzugt ist aber die Herstellung alleine durch Umformung, da hierdurch der Herstellaufwand gegenüber dem Stand der Technik reduziert werden kann.

Durch den Umformprozess wird das Material in seiner Dichte geändert. Insbesondere kann man in einem Schliff durch das Bauteil den Herstellprozess erkennen.

Ist das Bauteil durch ein zerspanendes Verfahren, wie z. B. Drehen, gemäß dem Stand der Technik hergestellt, so verlaufen die Struktur-/Fließlinien im Wesentlichen parallel und zeigen in die gleiche Richtung. Wird das Bauteil durch Stauchen bzw. Umformen in Form gebracht, d.h. die Ankippungen, wird durch ein Umformverfahren hergestellt, so sind die Strukturlinien durch den Umformprozess gebeugt. Durch einen metallurgischen Schliff kann somit auf das Herstellverfahren rückgeschlossen werden.

Dadurch, dass die Kraftlinien nach einem Umformen im Bauteil gebogen werden, erhöht man die Steifigkeit im Bauteil, was dazu führt, dass Montageschäden des Sensorbauteils im Bereich der so hergestellten Ankippung, insbesondere beim Aufschieben der Kraftstoffleitungen auf das Sensorbauteil sicherer als im Stand der Technik vermieden werden.

Als Materialien für das Sensorbauteilgehäuse finden bevorzugt Stahl, insbesondere Normalstahl, beispielsweise St35, St37, St38 sowie Edelstahl bzw. nichtrostende Stähle aufgrund ihrer hohen Korrosionsbeständigkeit oder NiFeCo-Legierungen beziehungsweise NiFe-Legierungen oder Kombinationen der zuvor angeführten Materialien Verwendung. Ganz bevorzugt ist aber die Verwendung von Edelstählen aufgrund der guten Korrosionsbeständigkeit des Werkstoffes, wobei hierfür kein zusätzlicher Platierungsprozess notwendig ist.

Neben der Vorrichtung gibt die Erfindung auch ein Verfahren gemäß Anspruch 4 zur Herstellung einer derartigen Vorrichtung an, das sich insbesondere gegenüber dem Verfahren zur Herstellung eines Sensorbauteilgehäuse mittels Dreh- und Fräsprozessen, wie in der US 2010/0186591 A1 offenbart, durch eine besonders einfache Herstellbarkeit auszeichnet.

Bei dem erfindungsgemäßen Verfahren wird zunächst ein Rohrabschnitt mit einer Rohrwandung zur Verfügung gestellt. Der Rohrabschnitt wird durch Abtrennen beispielsweise von einem geschweißten Rohr oder auch einem nahtlosen Rohr erhalten. Das Rohr umfasst eine Rohrwandung mit einer Wandstärke die im Bereich zwischen 0,5 mm und 1,0 mm liegt. Die Obergrenze der Wandstärke von beispielsweise 1,3 mm ist dadurch bedingt, dass stärkere Wandungen nur noch schwer durch Umformtechniken bearbeitet werden können, insbesondere wird der Kraftaufwand für einen Umfomprozess, wie beispielsweise das Stauchen zu groß. Die Untergrenze der Rohrwandstärke von beispielsweise 0,3 mm ist dadurch bedingt, dass Rohre mit zu geringer Wandstärke keine ausreichende Festigkeit für Umformprozesse aufweisen und knicken bzw. ausreißen können..

In die Rohrwandung wird eine Öffnung durch Lochen, für die Durchführung des Sensorbauteils eingebracht und beim ersten und/oder dem zweiten Ende des Rohrabschnittes durch einfaches Umformen eine Ankippung hergestellt, wobei sich der Durchmesser des Rohrabschnittes im Bereich des ersten und zweiten Endes verringert. Bei dem Umformprozess handelt es sich im Wesentlichen um einen Stauchprozess, bei dem durch ein Verjüngen die Ankippung hergestellt wird. Durch das erfindungsgemäße Verfahren lässt sich insbesondere eine hohe Materialeinsparung erzielen und eine kostengünstige Herstellung realisieren. Des Weiteren kann die Ankippung an den Rohrenden auf einfache Art und Weise durch Umformen hergestellt werden. Durch die Ankippung am Rohrende ist es möglich, das Sensorbauteilgehäuse an eine herkömmliche Kraftstoffleitung anzuschliessen, ohne dass die Dichtungen, beispielsweise die O-Ring-Dichtungen des Anschlusstückes bzw. Konnektors der Kraftstoffleitung beschädigt werden. Durch die erfindungsgemäße Ankippung werden also Undichtigkeiten im Bereich des Anschlusses der Kraftstoffleitung an das erfindungsgemäßen Sensorbauteilgehäuse vermieden.

Besonders bevorzugt ist es, wenn, um ein Überschieben der an das Sensorbauteilgehäuse angebrachten weiterführenden Leitungen zu verhindern, die Rohrwandung wiederum durch Umformen, insbesondere durch Stauchen mit einer Verdickung versehen wird.

Besonders bevorzugt ist es, wenn eine Durchführung, beispielsweise für ein im Sensorbauteilgehäuse liegendes Sensorbauteil, beispielsweise ein Innenrohr, in die Öffnung eingesetzt wird und auf einfache Art und Weise durch Hartlöten mit dem Sensorbauteilgehäuse verbunden wird.

Im Gegensatz hierzu erfolgte die Zentrierung bei der Vorrichtung gemäß der US 2010/0186591 A1 über den Innendurchmesser des Außenrohres, hier des Sensorbauteilgehäuses. Bei dieser Art der Zentrierung konnte aber keine Ankippung hergestellt werden, da eine Zentrierung mit Ankippung für eine derartige Art der Zentrierung äußerst schwierig war.

Die Erfindung soll nunmehr ohne Beschränkung hierauf anhand der Ausführungsbeispiele beschrieben werden.

Es zeigen:
- Fig. 1a - 1d: Erfindungsgemäße Vorrichtungen mit einem Sensorbauteilgehäuse, einer Durchführung sowie einem in dem Sensorbauteilgehäuse liegenden Sensorbauteil in Rohrform;
- Fig. 2: Vorrichtung gemäß dem Stand der Technik.
- Fig. 3a-3b: metallurgischer Schliff durch ein mittels Umformverfahren hergestelltes Bauteil beziehungsweise Teil eines Bauteiles
- Fig. 4a-4b: metallurgischer Schliff durch ein mittels zerspanendem Verfahren hergestellten Bauteiles beziehungsweise Teil eines Bauteiles

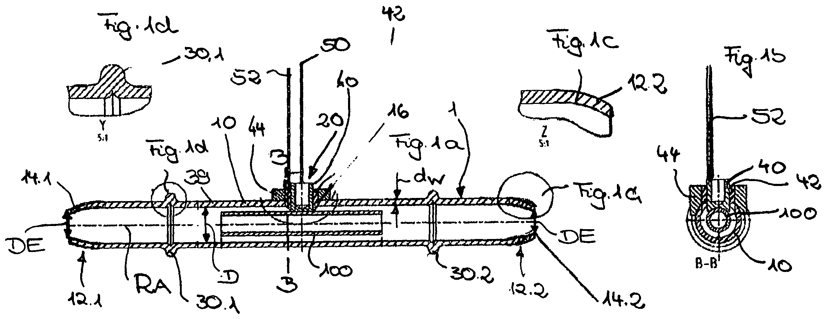

In den Figuren 1a bis 1d ist eine Vorrichtung zur Aufnahme und/oder Leitung eines flüssigen oder gasförmigen Mediums mit einem Sensorbauteil 100, das in ein Sensorbauteilgehäuse 10 eingelassen ist, gezeigt. Fig. 1a zeigt einen Längsschnitt durch eine derartige Vorrichtung 1. Das Sensorbauteilgehäuse 10 weist einen kreisrunden Querschnitt auf, der in der Ebene B-B in Fig. 1b dargestellt ist.

Der Durchmesser des kreisrunden Rohrquerschnitts beträgt D. Erfindungsgemäß ist vorgesehen, dass das Sensorbauteilgehäuse 10 im Bereich des ersten Endes 12.1 sowie des zweiten Endes 12.2 eine Ankippung 14.1 bzw. 14.2 aufweist. Die Ankippung 12.2 ist in Fig. 1c näher dargestellt. Bei der Ankippung wird das erste Ende 12.1 bzw. das zweite Ende 12.2 des Rohrabschnittes des Sensorbauteilgehäuses 10 gestaucht, beispielsweise mit einem Umformwerkzeug der Durchmesser vom Durchmesser D im Bereich der Rohrmitte auf den Durchmesser DE am Rohrende (12.1, 12.2) verringert. Obwohl vorliegend der Durchmesser DE am ersten Ende 12.1 den Durchmesser DE am zweiten Ende 12.2 entspricht, muss dies nicht zwangsläufig so sein, vielmehr wären auch unterschiedliche Durchmesser möglich.

Die Wandstärke der Rohrwandung 39 beträgt d_{w}. Erfindungsgemäß liegt die Stärke der Rohrwandung 39 im Bereich 0,5 mm bis 1 mm.

In die Rohrwandung 39 der Wandstärke d_{W} des Sensorbauteilgehäuses 10 ist in vorliegendem Ausführungsbeispiel eine Öffnung 16 eingebracht, die vorteilhaft durch Lochung durch die Wandstärke d_{W} der Rohrwandung 39 hindurch hergestellt wird. In die durch Lochung hergestellte Öffnung 16 ist in dem vorliegenden Ausführungsbeispiel eine Glas-Metall-Durchführung eingebracht. Die Glas-Metall-Durchführung umfasst ein Gehäuseteil, das vorliegend, aber nicht ausschließlich in Form eines Bechers 40 ausgebildet ist, wie beispielsweise in der US 2010/0186501 A1 beschrieben, deren Offenbarungsgehalt in vorliegende Anmeldung voll umfänglich mit aufgenommen wird. Der Becher 40 ist bevorzugt ein Metallteil, das in die Öffnung 16 der Rohrwandung 39 eingeglast ist. Das Glasmaterial zwischen dem Becher 40 und der Öffnung 16 ist mit 42 gekennzeichnet. Da die Wandstärke d_{W} des Rohrs für die Einglasung nicht ausreichend ist, umfasst die Durchführung gemäß der Erfindung in einer bevorzugten Ausführungsform ein auf die Rohrwandung 39 aufgesetztes Verstärkungsbauteil 44. Das Verstärkungsbauteil 44 kann das gleiche Material wie die Rohrwandung 39 aufweisen, muss aber nicht. Bevorzugt wird nämlich das Material des Sensorbauteiles derart gewählt, dass es abgestimmt ist auf das das Sensorbauteil durchströmende oder zu detektierende Medium. Das Material des Verstärkungsbauteil ist abgestimmt auf eine stabile, sichere und optimale Einglasung mit Glas- bzw. Glaskeramikmaterial 42. Das zusätzliche Verstärkungsbauteil 44 wird bevorzugt durch Hartlöten mit der Rohrwandung 39 verbunden. In den eingeglasten Becher 40 der Durchführung wird eine erste Leitung 50 z. B. für einen Temperatursensor (nicht gezeigt) eingeführt. Der Becher 40 der Durchführung ist wiederum mit dem Sensorbauteil 100 in Form eines Innenrohres leitend verbunden, wie in der US 2010/0186501 A1 beschrieben. Der Becher 40 ist mit einer Leitung 52 verbunden, die auf ein Potential gelegt werden kann. Zwischen der Rohrwandung 39 des Sensorbauteilgehäuses und dem Innenrohr lässt sich dann eine Potentialdifferenz bzw. eine Spannung anlegen, wenn die Rohrwandung 39 bspw. geerdet ist und der Becher 40 und damit das Innenrohr 100 auf Potential liegt. Die Potentialdifferenz zwischen Außenrohr und Innenrohr ist Basis für eine kapazitive

Messung von durch das Innenrohr hindurchströmender Flüssigkeit oder Gas. Aus den Kapazitätswerten bzw. den Änderungen der kapazitiven Werte können dann Rückschlüsse darauf gezogen werden, welches Material bzw. Medium durch das rohrförmige Sensorbauteil 100 hindurchströmt. Durch die Ausgestaltung des Sensorbauteiles 100 als Innenrohr mit parallelen Flächen zum Außenrohr werden die Kapazitätenflächen stark vergrößert und kapazitive Messungen von strömenden Medien in rohrförmigen Sensorabschnitten verbessert. Als Medium, das mit Hilfe der in Figur 1a dargestellten Vorrichtung vermessen werden kann, sind die nachfolgenden Medien:
Erdgas, Wasserstoff, Stickstoff, Sauerstoff, Abgase von Verbrennungsmotoren, industrielle Prozessgase, Autogase, Luft, Wasser, insbesondere Salzwasser, Öle, insbesondere für Motoren, Getriebe- und Hydraulikanwendungen, Alkohole, insbesondere Methanol und Ethanol, Benzin- und Dieselkraftstoff, Rapsöl, Methylesther, Flugzeugturbinentreibstoffe, Harnstoffe und Harnstofflösungen, Fluorwasserstoffe
denkbar.

Um ein Überschieben von an das erste und das zweite Ende 12.1, 12.2 angeschlossenen Kraftstoffleitungen zu verhindern, weist die Rohrwandung 39 des Rohrs des Sensorbauteilgehäuses umlaufende Verdickungen 30.1, 30.2 auf. Die umlaufende Verdickung 30.1 ist in Figur 1d näher dargestellt.

Wie aus Figur 1d hervorgeht, kann die Verdickung unter Beibehaltung des Rohrdurchmessers ebenfalls durch Stauchen der Rohrwandung auf sehr einfache Art und Weise hergestellt werden

In Figur 1b ist ein Querschnitt durch eine Vorrichtung gemäß Fig. 1a entlang der Linie B-B dargestellt.

Deutlich zu erkennen ist die Leitung 52, die an den Becher 40 leitend angeschlossen ist, der wiederum mit dem als Innenrohr ausgeführten Sensorbauteil verbunden ist, so dass ein Potential am Sensorbauteil 100 angelegt werden kann. Der Becher 40 der Durchführung ist in die Öffnung und das auf die Öffnung aufgesetzte zusätzliche Bauteil beziehungsweise Verstärkungsbauteil 44 mit einem Glasmaterial 42 eingeglast. Hierdurch wird die Leitung 52 elektrisch von der Rohrwandung 10 getrennt. Die Rohrwandung 10 kann beispielsweise geerdet werden, so dass eine kapazitive Messung zwischen dem Sensorbauteil 100 als Innenrohr und dem Sensorbauteilgehäuse 10 in Rohrform , d. h. dem Außenrohr 10 möglich ist

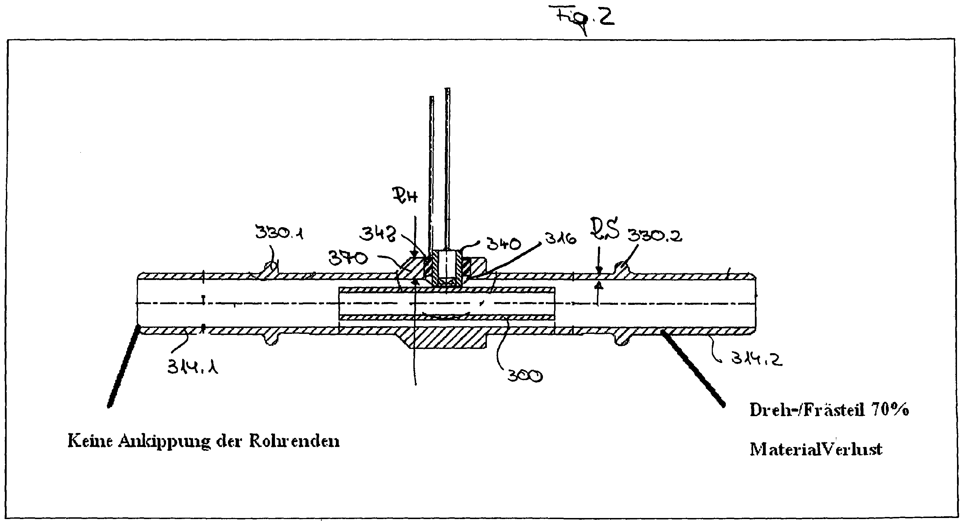

Fig. 2 zeigt eine Ausführungsform einer rohrförmigen Vorrichtung zur Aufnahme oder Leitung von gasförmigen oder flüssigen Medien mit einem Sensorbauteilgehäuse 310 und einem Sensorbauteil 300 gemäß dem Stand der Technik, wie in der US 2010/0186501 A1 beschrieben, deren Offenbarungsgehalt in vorliegende Anmeldung mit eingeschlossen wird. Gleiche Bauteile wie in den Fig. 1a - 1d werden mit einer um 200 erhöhten Bezugsziffer belegt. Im Gegensatz zur Ausgestaltung gemäß den Fig. 1a - 1d ist beim Stand der Technik das Sensorbauteilgehäuse aus einem Stück gefräst und gedreht. Ein Becher 340 der leitend mit dem als Sensorbauteil ausgebildeten Innenrohr 300 verbunden ist, ist in eine in die Rohrwand eingelassene Öffnung 316 eingeglast. Damit liegt das Glasmaterial 342 direkt zwischen einer Erhebung 370 der Rohrwandung und dem Becher 340 an. Die Einglasung ist mit Bezugsziffer 342 gekennzeichnet. Die Dicke der Rohrwand beträgt RS, die Dicke der Erhebung 370 beträgt RH. In vorliegendem Fall ist die Stärke der Rohrwand RS geringer als die der Erhebung 370. Dies wird dadurch erreicht, dass aus einem Rohling mit einer Dicke RH durch spanende Bearbeitung ein Rohr mit der Dicke RS für die Rohrwandung hergestellt wird, was aufwändig und mit hohem Materialverbrauch verbunden ist. Durch die spanende Bearbeitung werden auf der Rohrwandung des Sensorbauteilgehäuses 310 Verdickungen 330.1, 330.2 zur Verfügung gestellt. Im Gegensatz zur Erfindung können an den Enden 314.1, 314.2 des Sensorbauteilgehäuses 310 keine Ankippung, die sich über einen längeren Abschnitt erstrecken, zur Verfügung gestellt, womit kein dichter Anschluss einer Anschlussleitung insbesondere einer Fluid- bevorzugt einer Kraftstoffleitung möglich ist. Über das direkte Einglasen in die Rohrwandung hinaus zeichnet sich die Vorrichtung gemäß Fig. 2 auch dadurch aus, dass der Becher 340 in direkter Verbindung mit einem als Sensorbauteil ausgebildeten Innenrohr 300 steht. Das Innenrohr 300 hat eine sehr geringe Wandstärke, bevorzugt eine Wandstärke im Bereich 0,3 mm bis 1,0 mm. Das Innenrohr 300, das mit dem Sensorabschnitt verbunden ist, erstreckt sich über wenigstens die halbe Länge L des als Rohr ausgebildeten Sensorbauteilgehäuses 310. Durch das Einbringen des Innenrohres 300 können verschiedene Vorteile erreicht werden. So sorgt das Innenrohr dafür, dass eine laminare Strömung in dem rohrförmigen Sensorbauteil vorliegt und es zu keiner Strömungszerstörung kommt. Des Weiteren werden Wärmeflächen, die in direktem Kontakt mit einem im Becher angeordneten Temperaturfühler (nicht gezeigt) stehen, wesentlich erweitert bzw. vergrößert. Dies wiederum verbessert die Genauigkeit von Temperaturmessungen von Medien, die durch das rohrförmige Sensorbauteil hindurchströmen. Für eine kapazitive Messung wird das Außenrohr auf ein Potential gelegt und das Innenrohr auf ein anderes Potential. Auf diese Art und Weise wird es möglich, eine kapazitive Messung vorzunehmen, die Aufschluss über die Zusammensetzung des durch die Vorrichtung hindurchtretende Mediums gibt. Durch die Ausgestaltung des Innenrohrs mit parallelen Flächen zum Außenrohr werden die kapazitiven Flächen stark vergrößert und die kapazitive Messung von strömendem Medium in der Vorrichtung verbessert. Die Abmessungen des Innenrohres wie im Stand der Technik beschrieben können auch für das in der Erfindung eingesetzte Sensorbauteil 100 (in Fig. 1a - 1d) in Form eines Innenrohres gewählt werden mit den zuvor beschriebenen Vorteilen.

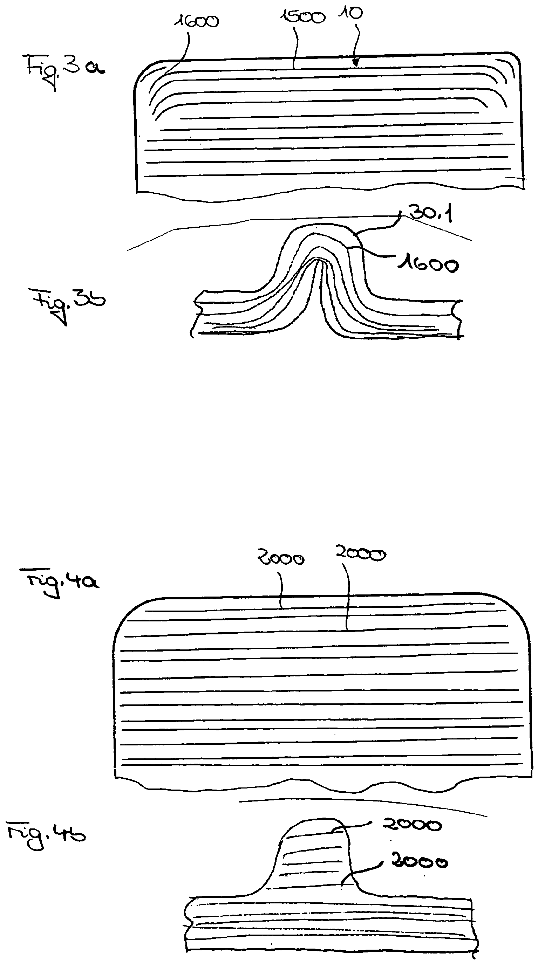

In Fig. 3a-3b sind Prinzipbilder eines metallurgischer Schliff durch einen Ausschnitt eines mit einem Umformverfahren hergestellten erfindungsgemäßen Bauteil 10, wie in den Fig. 1a - 1d dargestellt, gezeigt. Die Prinzipbilder sind nicht maßstäblich und soll nur prinzipiell den Verlauf von Fließlinien bei einem umgeformten Bauteil zeigen gleiche Bauteile wir in Fig. 1a - 1d sind mit denselben Bezugsziffern bezeichnet.

Wie aus dem metallurgischen Schliff gemäß Fig. 3a-3b hervorgeht, sind die nach dem erfindungsgemäßen Verfahren hergestellten Bauteil durch Struktur-/Fließlinien 1500 gekennzeichnet, die im Bereich 1600 durch den Umformprozess gebeugt worden sind. Besonders prominent ist die Beugung der Struktur-/Fließlinien für die umlaufende Verdickung 30.1, 30.2 wie in Figur 3b gezeigt, die durch den Umformprozess im Bereich 1600 gebeugt sind.

Im Gegensatz hierzu zeigen Fig. 4a-4b Prinzipbilder eines metallurgischen Schiffes eines mittels Zerspanen hergestelltes Bauteil 300 gemäß der US 2010/0186501 A1, das insbesondere ein Drehteil ist. Wiederum eingezeichnet sind die Struktur-/Fließlinien 2000. Die Struktur-/Fließlinien 2000 sind im Wesentlichen parallel und zeigen in die gleiche Richtung. Auch dieses Prinzipbild ist nicht maßstäblich und soll nur prinzipiell den Verlauf von Fließlinien bei einem durch Drehen und Fräsen hergestellten Bauteil zeigen. Besonders deutlich ist wieder der Unterschied im Verlauf der Struktur-/Fließlinien 2000 im Bereich einer Verdickung gemäß Figur 4b. Der Unterschied zum umgeformten Bauteil gemäß Figur 3b ist deutlich.

Mit der Erfindung wird eine gegenüber dem Stand der Technik in Form der US 2010/0186501 A1 einfache und mit weniger Materialverlust herzustellende Vorrichtung zur Bestimmung der Zusammensetzung einer durch ein rohrförmiges Sensorbauteilgehäuse, das mit einem Sensorbauteil versehen ist, hindurchgeleitetes flüssiges oder gasförmiges Medium zur Verfügung gestellt. Die Ankippung, die auf einfache Art und Weise durch ein Verfahren, das wenigstens einen Umformprozess umfasst, hergestellt wird, ermöglicht einen dichten Anschluss an Fluid- insbesondere Kraftstoffleitungen. Durch die Herstellung der Ankippung mit Hilfe eines Umformprozesses kann gegenüber z. B. einer rein spanenden Bearbeitung eine erhebliche Materialeinsparung erzielt werden.Das durch ein Verfahren, umfassend wenigstens einen Umformprozess, hergestellte Bauteil weist im Schnitt durch die Umformung bedingte gebeugte Struktur-/Fließlinien auf, im Gegensatz zu parallel verlaufenden Flusslinien bei nach spanenden Verfahren hergestellten Bauteilen. Hierdurch kann vorteilhaft ein umgeformtes Bauteil im Bereich der Ankippung eine höhere Steifigkeit als ein spanend bearbeitetes Bauteil aufweisen, wodurch Materialschäden, z. B. beim Aufschieben einer Kraftstoffleitung auf die Ankippung, vermieden werden. Ein weiterer Vorteil der Erfindung ist darin zu sehen, dass das Sensorbauteilgehäuse und das Verstärkungsbauteil in einer bevorzugten Ausführungsform aus unterschiedlichen Materialien hergestellt werden können, wobei das Material des Sensorbauteilgehäuses an das zu detektierende Medium angepasst ist und das Material des Verstärkungsbauteiles auf eine stabile, sichere und optimale Einglasung abgestimmt ist.

## Patentansprüche

1. Vorrichtung zur Aufnahme und/oder Leitung eines flüssigen oder gasförmigen Mediums mit einem Sensorbauteilgehäuse (1) und einem Sensorbauteil (100), das in ein Sensorbauteilgehäuse (1) eingelassen ist, wobei das Sensorbauteilgehäuse (1) wenigstens einen Rohrabschnitt mit einer Rohrwandung (39) mit einer Öffnung (16) umfasst und
- der Rohrabschnitt einen Durchmesser (D) sowie ein erstes und ein zweites Ende (12.1, 12.2) aufweist;
- die Rohrwandung eine Wandstärke im Bereich 0,5 mm bis 1 mm aufweist und das Sensorbauteilgehäuse eine Durchführung für ein Sensorbauteil umfasst, wobei die Öffnung (16) die Durchführung für das Sensorbauteil aufnimmt und die Durchführung, ein Glas- oder Glaskeramikmaterial (42) und ein Bauteil (44) als Verstärkungsbauteil aus einem Material, das auf eine stabile und optimale Einglasung mit dem Glas- oder Glaskeramikmaterial 42 abgestimmt ist, umfasst, wobei das Bauteil (44), als Verstärkungsbauteil mit der Rohrwandung (39) durch eine Lot- oder Schweißverbindung verbunden ist, wobei
- das erste und/oder das zweite Ende (12.1, 12.2) Ankippungen (14.1, 14.2) aufweist, so dass sich der Durchmesser (D) zum ersten und/oder zweiten Ende (12.1, 12.2) des Rohrabschnitts hin auf einen Enddurchmesser (DE) verjüngt;
- die Ankippungen durch einen Umformprozess, insbesondere Stauchen, hergestellt werden;
- die Rohrwandung (39) des Weiteren wenigstens eine durch Umformen hergestellte Verdickung (30.1, 30.2) aufweist; und
- das Sensorbauteil (100) ein Innenrohr für eine kapazitive Messung ist und
das Verstärkungsbauteil (44) die im Bereich der Durchführung für das Sensorbauteil benötigte Wandstärke für eine Einglasung zur Verfügung stellt.

2. Vorrichtung nach Anspruch 1,
**dadurch gekennzeichnet, dass**
das Sensorbauteilgehäuse (1) einem der nachfolgenden Materialien der nachfolgenden Materialien oder Kombinationen hiervon besteht:
Stahl,
Edelstahl,
NiFeCo-Legierungen,
NiFe-Legierungen.

3. Vorrichtung nach einem der Ansprüche 1 bis 2,
**dadurch gekennzeichnet, dass**
das flüssige oder gasförmige Medium insbesondere eines der nachfolgenden Medien ist:
Erdgas, Wasserstoff, Stickstoff, Sauerstoff, Abgas von Verbrennungsmotoren, industrielle Prozessgase, Autogas, Luft, Wasser, insbesondere Salzwasser, Öle, insbesondere für
Motoren,
Getriebe- und Hydraulikanwendungen,
Alkohole,
insbesondere Methanol und Ethanol,
Benzin- und Dieselkraftstoff,
Rapsöl,
Methylesther,
Flugzeugturbinentreibstoffe,
Harnstoffe und Harnstofflösungen,
Flurkohlenwasserstoffe.

4. Verfahren zur Herstellung einer Vorrichtung gemäß einem der Ansprüche 1 bis 3, wobei
- ein Rohrabschnitt mit einem Durchmesser (D) und einer Rohrwandung (39) für ein Sensorbauteilgehäuse zur Verfügung gestellt wird;
- in die Rohrwandung (39) wenigstens eine Öffnung (16) zur Aufnahme einer Durchführung für ein als Innenrohr für eine kapazitive Messung ausgebildetes Sensorbauteil, insbesondere durch Lochen, eingebracht wird; wobei
- an dem ersten und/oder dem zweiten Ende (12.1, 12.2) des Rohrabschnittes durch Umformen, insbesondere Stauchen, eine Ankippung (14.1, 14.2) und eine Verdickung (30.1, 30.2) hergestellt wird, derart, dass sich der Durchmesser (D) des Rohrabschnittes im Bereich der Ankippung verringert und die Verdickung derart ausgebildet ist, dass ein Überschieben von an das erste und/oder zweite Ende (12.1, 12.2) angeschlossenen Leitungen verhindert wird und
- das Sensorbauteil in Form eines Innenrohres für eine kapazitive Messung in das Innere des Sensorbauteilgehäuses eingebracht wird.

5. Verfahren nach Anspruch 4,
**dadurch gekennzeichnet, dass**
die Durchführung für das Sensorbauteil (100) mit der Rohrwandung (39) verbunden wird.

## Claims

1. Device for receiving and/or conducting a liquid or gaseous medium, comprising a sensor component housing (1) and a sensor component (100) which is inserted in a sensor component housing (1), wherein the sensor component housing (1) comprises at least one tube section having a tube wall (39) with an opening (16), and
- the tube section has a diameter (D) and also a first and a second end (12.1, 12.2);
- the tube wall has a wall thickness in the range from 0.5 mm to 1 mm and the sensor component housing comprises a feedthrough for a sensor component, wherein the opening (16) receives the feedthrough for the sensor component and the feedthrough comprises a glass or glass-ceramic material (42) and a component (44) as a reinforcing component made of a material which is suitable for stable and optimal bonding by glass using the glass or glass-ceramic material 42, wherein the component (44) as a reinforcing component is connected to the tube wall (39) by a soldered or welded joint, wherein
- the first and/or the second end (12.1, 12.2) has sloping regions (14.1, 14.2) such that the diameter (D) tapers to a final diameter (DE) at the first and/or second end (12.1, 12.2) of the tube section;
- the sloping regions are produced by a shaping process, in particular by compression;
- the tube wall (39) additionally has at least one thickened region (30.1, 30.2) produced by shaping; and
- the sensor component (100) is an inner tube for a capacitive measurement, and
the reinforcing component (44) provides the wall thickness necessary for bonding by glass in the area of the feedthrough for the sensor component.

2. Device according to claim 1,
**characterized in that**
the sensor component housing (1) is made one of the following materials of the following materials or combinations thereof:
steel,
stainless steel,
NiFeCo alloys,
NiFe alloys.

3. Device according to any one of claims 1 to 2,
**characterized in that**
the liquid or gaseous medium is in particular one of the following media:
natural gas, hydrogen, nitrogen, oxygen, exhaust gas from internal combustion engines, industrial process gasses, liquefied petroleum gas, air, water, in particular salt water, oils, in particular for
engines,
gearbox and hydraulic applications,
alcohols,
in particular methanol and ethanol,
petrol and diesel fuel,
rapeseed oil,
methyl esters,
aircraft engine fuels,
ureas and urea solutions,
hydrofluorocarbons.

4. Method for producing a device according to any one of claims 1 to 3,
wherein
- a tube section having a diameter (D) and a tube wall (39) is provided for a sensor component housing;
- at least one opening (16) for receiving a feedthrough for a sensor component configured as an inner tube for a capacitive measurement is created in the tube wall (39), in particular by punching;
wherein
- a sloping region (14.1, 14.2) and a thickened region (30.1, 30.2) is produced at the first and/or second end (12.1, 12.2) of the tube section by shaping, in particular by compression, such that the diameter (D) of the tube section is reduced in the area of the sloping region and the thickened region is formed such that lines connected to the first and/or second end (12.1, 12.2) cannot be pushed therebeyond, and
- the sensor component in the form of an inner tube for a capacitive measurement is introduced into the interior of the sensor component housing.

5. Method according to claim 4,
**characterized in that**
the feedthrough for the sensor component (100) is connected to the tube wall (39).

## Revendications

1. Dispositif pour recevoir et/ou conduire un milieu liquide ou gazeux, comportant un boîtier de composant capteur (1) et un composant capteur (100) noyé dans un boîtier de composant capteur (1), dans lequel le boîtier de composant capteur (1) comprend au moins une section de tube présentant une paroi de tube (39) avec une ouverture (16) et
- la section de tube présente un diamètre (D) et une première et une deuxième extrémité (12.1, 12.2) ;
- la paroi de tube présente une épaisseur de paroi dans la plage comprise entre 0,5 mm et 1 mm et le boîtier de composant capteur comprend un passage pour un composant capteur, l'ouverture (16) recevant le passage pour le composant capteur et le passage comprenant un matériau en verre ou en vitrocéramique (42) et un composant (44) utilisé comme composant de renforcement dans un matériau qui est adapté à une vitrification stable et optimale avec le matériau en verre ou en vitrocéramique (42), le composant (44) utilisé comme composant de renforcement étant relié à la paroi de tube (39) par une brasure ou une soudure, dans lequel
- la première et/ou la deuxième extrémité (12.1, 12.2) présentent des inclinaisons (14.1, 14.2), de sorte que le diamètre (D) se rétrécit vers la première et/ou la deuxième extrémité (12.1, 12.2) de la section de tube à un diamètre final (DE) ;
- les inclinaisons sont produites par un processus de formage, en particulier par refoulement ;
- la paroi de tube (39) présente en outre au moins un épaississement (30.1, 30.2) produit par formage ; et
- le composant capteur (100) est un tube intérieur pour une mesure capacitive et
- le composant de renforcement (44) fournit l'épaisseur de paroi nécessaire pour une vitrification dans la zone du passage pour le composant capteur.

2. Dispositif selon la revendication 1,
**caractérisé en ce que**
le boîtier de composant capteur (1) est constitué de l'un des matériaux suivants ou de combinaisons de ceux-ci :
acier,
acier inoxydable,
alliages NiFeCo,
alliages NiFe.

3. Dispositif selon l'une des revendications 1 à 2,
**caractérisé en ce que**
le milieu liquide ou gazeux est en particulier l'un des milieux suivants :
gaz naturel, hydrogène, azote, oxygène, gaz d'échappement de moteurs à combustion interne, gaz de processus industriels, GPL, air, eau, en particulier eau salée, huiles, en particulier pour
moteurs,
applications de transmission et hydrauliques,
alcools,
en particulier méthanol et éthanol,
essence et gazole,
huile de colza,
ester de méthyle,
carburant pour turbines d'avion,
urée et solutions d'urée,
hydrocarbures fluorés.

4. Procédé de fabrication d'un dispositif selon l'une des revendications 1 à 3, dans lequel
- une section de tube présentant un diamètre (D) et une paroi de tube (39) pour un boîtier de composant capteur est fournie ;
- au moins une ouverture (16) est ménagée, en particulier par perforation, dans la paroi de tube (39) pour recevoir un passage pour un composant capteur réalisé sous la forme d'un tube intérieur pour une mesure capacitive, dans lequel
- une inclinaison (14.1, 14.2) et un épaississement (30.1, 30.2) sont réalisés à la première et/ou la deuxième extrémité (12.1, 12.2) de la section de tube par formage, en particulier par refoulement de telle sorte que le diamètre (D) de la section de tube est réduit dans la zone de l'inclinaison, et l'épaississement est réalisé de telle sorte qu'un chevauchement de conduites raccordées à la première et/ou la deuxième extrémité (12.1, 12.2) soit empêché et
- le composant capteur est introduit à l'intérieur du boîtier de composant capteur sous la forme d'un tube intérieur pour une mesure capacitive.

5. Procédé selon la revendication 4,
**caractérisé en ce que**
le passage pour le composant capteur (100) est relié à la paroi de tube (39).
